# EUROPÄISCHE PATENTANMELDUNG

(11) **EP 0 956 779 A1**
(43) Veröffentlichungstag der Anmeldung: **17.11.1999**
(21) Anmeldenummer: 98108520.2
(22) Anmeldetag: 11.05.1998
(51) Int. Cl.: A23L 1/035

(54) **Nahrungsmittel, welche wasserunlösliche Komponenten enthalten**

(71) Anmelder: VESIFACT AG, 6340 Baar (CH)
(72) Erfinder: Weder, Hans Georg, 8803 Rüschlikon (CH); Weder, Marc Antoine, 8803 Rüschlikon (CH); Schneider, Martin, 6314 Unterägeri (CH); Supersaxo, Andreas, 6340 Baar (CH)
(74) Vertreter: Ryffel, Rolf

(57) **Zusammenfassung**

Beschrieben wird die Verwendung eines NanoFood, enthaltend als präparative Zusammensetzung
(a) ein membranbildendes Molekül,
(b) einen Coemulgator und
(c) einen lipophilen Bestandteil
in Nahrungs-Endprodukten.

## Beschreibung

Die vorliegende Erfindung betrifft die Verwendung von NanoFood in Nahrungs-Endprodukten, Nahrungs-Endprodukte, enthaltend diese NanoFood sowie die verschiedenen Verwendungen dieser Nahrungs-Endprodukte für Mensch und Tier.

Nahrungs-Endprodukte im Sinne der vorliegenden Erfindung sind Getränke, Nahrungsmittel, Diätetika, Energy Drinks, Energy Food und Functional Food. Functional Food ist jede modifizierte Nahrung oder Bestandteile davon, die zusätzlich zu Ihrer natürlichen Form einen präventiven oder gesundheitlichen Nutzen ausweisen sowie Körperfunktionen stimulieren oder verbessern. Synonyme Begriffe für Functional Food sind Pharmafood, Medical Food oder Designer Food.

Der Erfindung liegt die Aufgabe zugrunde, schwer wasserlösliche oder wasserunlösliche Nährstoffe und Nutraceuticals stabil in Nahrungs-Endprodukten einzuarbeiten. Nutraceuticals sind natürliche oder synthetische Substanzen, die einen präventiven oder gesundheitlichen Nutzen ausweisen sowie Körperfunktionen stimulieren oder verbessern.

Zur Lösung dieser Aufgabenstellung stehen im wesentlichen folgende zwei Methoden zur Verfügung: Mikroverkapseln von Feststoffen und Oelen und Emulgieren von Oelen oder von in Oel suspendierten, gegebenenfalls mikronisierten Feststoffen. Diese Herstellungsprozesse sind sehr aufwendig und schwer kontrollierbar. Solche in Endprodukte eingearbeiteten mikroverkapselten oder emulgierten Nährstoffe und Nütraceuticals können sedimentieren oder aufrahmen, was eine unerwünschte Qualitätsverminderung zur Folge hat. Beide Phänomene könnten reduziert oder verhindert worden, gelänge es, ultra kleine, homogene Nährstoff oder Nutraceutcial beladene Partikel herzustellen, die in Wasser oder in der wässrigen Phase des Endproduktes suspendiert oder emulgiert praktisch nicht mehr aggregieren.

Ueberraschenderweise wurde gefunden, dass mit sogenannten NanoFood, enthaltend
(a) ein membranbildendes Molekül
(b) einen Coemulgator
(c) einen lipophilen Bestandteil,
wasserunlösliche oder schwer wasserlösliche Nährstoffe und Nutraceuticals in Nahrungs-Endprodukte eingebracht werden können, so dass deren Stabilität gewährleistet ist.

Gegenstand der vorliegenden Erfindung ist daher die Verwendung eines NanoFood, enthaltend als präparative Zusammensetzung
(a) ein membranbildendes Molekül
(b) einen Coemulgator und
(c) einen lipophilen Bestandteil,
in Nahrungs-Endprodukten.

Vorzugsweise enthält das NanoFood
(a) als membranbildende Moleküle Substanzen, die geeignet sind, Zweischichtsysteme (sog. "Bilayer") auszubilden,
(b) als Coemulgatoren Substanzen, die bevorzugt O/W Strukturen ausbilden und
(c) als lipophile Komponente einen gebräuchlichen lipophilen Nährstoff oder ein gebräuchliches lipophiles Nutraceutical.

Vorzugsweise enthält das NanoFood als Komponente (a) ein natürliches Phospholipid, ein hydriertes oder teilhydriertes Phospholipid, ein Lysophospholipid oder Mischungen aus diesen Verbindungen.

Ganz besonders bevorzugt ist dabei ein Phospholipid der Formel 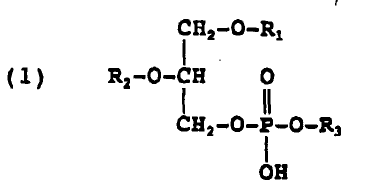 worin
- R₁: C₁₀-C₂₀-Acyl;
- R₂: Wasserstoff oder C₁₀-C₂₀-Acyl
- R₃: Wasserstoff, 2-Trimethylamino-1-ethyl, 2-Amino-1-ethyl, nicht substituiertes oder durch eine oder mehrere Carboxy-, Hydroxy- oder Amino-Gruppen substituiertes C₁-C₃-Alkyl; die Inositol- oder die Glycerylgruppe bedeuten, oder Salze dieser Verbindungen.

C₁₀-C₂₀-Acyl ist vorzugsweise ein geradkettiges C₁₀-C₂₀-Alkanoyl mit einer geraden Anzahl an C-Atomen und geradkettiges C₁₀-C₂₀-Alkenoyl mit einer oder mehreren Doppelbindungen und einer geraden Anzahl an C-Atomen.

Geradkettiges C₁₀-C₂₀-Alkanoyl mit einer geraden Anzahl an C-Atomen sind beispielsweise n-Dodecanoyl, n-Tetradecanoyl, n-Hexadecanoyl oder n-Octadecanoyl.

Geradkettiges C₁₀-C₂₀-Alkenoyl mit einer Doppelbindung und einer geraden Anzahl an C-Atomen sind beispielsweise 6-cis- oder-6-trans-, 9-cis- oder 9-trans- Dodecenoyl, -Tetradecenoyl, -Hexadecenoyl, -Octadecenoyl oder -Icosenoyl, insbesondere 9-cis-Octa-decenoyl (Oleoyl), ferner 9,12-cis-Octadecadienoyl oder 9,12,15-cis-octadecatrienoyl.

Ein Phospholipid der Formel (1), worin R₃ 2-Trimethylamino-1-ethyl bedeutet, wird mit dem Trivialnamen Lecithin und ein Phospholipid der Formel (1), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen oder Mischungen davon.

Das Phospholipid der Formel (1) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter vorn definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ von natürlich vorkommenden Fettsäuregemischen abgeleitet sind.

Die Forderung "im wesentlichen reines" Phospholipid der Formel (1) definiert einen Reinheitsgrad von mehr als 90 Gew.-%, vorzugsweise mehr als 95 Gew.-% des Phospholipids der Formel (1), welcher anhand geeigneter Bestimmungsme-. thoden, z.B. papier- oder dünnschichtchromatographisch, mit HPLC oder enzymatischem Farbtest, nachweisbar ist.

In einem Phospholipid der Formel (1) ist R₃ mit der Bedeutung C₁-C₄-Alkyl beispielsweise Methyl oder Ethyl. Die Bedeutung Methyl ist bevorzugt.

R₃ mit den Bedeutungen durch eine oder mehrere Carboxy-, Hydroxy- oder Amino-Gruppen substituiertes C₁-C₅-Alkyl sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl, 3-Amino-3-carboxy-n-propyl oder 2-Amino-2-carboxy-n-propyl, vorzugsweise 2-Amino-2-carboxyethyl.

Phospholipide der Formel (1) mit diesen Gruppen können in Salzform, z.B. als Natrium- oder Kaliumsalz, vorliegen.

Phospholipide der Formel (1), worin R₃ die Inositol- oder die Glycerylgruppe bedeutet, sind unter den Bezeichnungen Phosphatidylinositol und Phosphatidylglycerol bekannt.

Für die Acylreste in den Phospholipiden der Formel (1) sind auch die in Klammern angegebenen Bezeichnungen gebräuchlich:
9-cis-Dodecenoyl (Lauroleoyl), 9-Cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-Icosenoyl (Gadoleoyl), 5,8,11,14-cis-Eicosatetraenoyl (Arachidonoyl), n-Dodecanoyl, (Lauroyl), n-Tetradecanoyl (Myristoyl), n-Hexadecanoyl (Palmitoyl), n-Octadecanoyl (Stearoyl), n-Icosanoyl (Arachidoyl), n-Docosanoyl (Behenoyl), n-Tetracosanoyl (Lignoceroyl).

Ein Salz des Phospholipids der Formel (1) ist annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen der Qualität LI-POID S 100 oder S 75 oder ein Lecithin definiert in der Monographie der USP23 / NF 18.

Die Komponente (a) wird vorzugsweise in einer Konzentration von ca. 0,1 bis 30 Gew.-%, bezogen auf das Gesamtgewicht der Komponenten (a), (b) und (c), eingesetzt.

Als Komponente (b) wird vorzugsweise ein Emulgator oder Emulgatormischungen verwendet, der/die bevorzugt O/W-Strukturen ausbildet(n).

Speziell bevorzugte Emulgatoren sind:
- Alkali-, Ammonium- und Aminiumsalze von Fettsäuren. Beispiele für solche Salze sind Lithium-, Natrium-, Kalium-, Ammonium-, Triethylamin-, Ethanolamin-, Diethanolamin- oder Triethanolaminsalze. Insbesondere werden die Natrium-, Kalium- oder Ammonium-(NR₁R₂R₃)-salze verwendet, wobei R₁, R₂ und R₃ unabhängig voneinander Wasserstoff, C₁-C₄-Alkyl oder C₁-C₄-Hydroxyalkyl bedeuten.
- Alkylsulfate wie z.B. Natriumdocecylsulfat
- Salze der Gallensäure wie z.B. Natriumcholat, Natriumglycocholat und Natriumtaurocholat;
- Partialfettsäureester des Sorbitans wie z.B. Sorbitanmonolaurat;
- Zuckerester wie z.B. Sucrosemonolaurat;
- Fettsäurepartialglyceride wie z.B. Laurinsäuremonoglycerid;
- Polyglycerinester von Fettsäuren;
- Propylenglycolester von Fettsäuren;
- Milchsäureester von Fettsäuren wie z.B. Natriumstearoyl-lactyl-2-lactat
- Proteine wie z.B. Kasein.

Emulgatoren vom Polyoxyethylen Typ sind ganz besonders bevorzugt. Beispiele solcher Emulgatoren sind:
- Polyethoxylierte Sorbitanfettsäureester wie z.B. Polysorbat 80;
- Polyethoxyliertes Vitamin E Derivate wie z.B. Vitamin E Polyethylen Glycol 1000 Succinat;
- Polyethoxylierte Fettsäurepartialglyceride wie z.B. Diethylenglykolmonostearat;
- Polyethoxylierte Kohlenhydrate;
- Blockpolymerisate von Ethylenoxid und Propylenoxid, wie z.B. Poloxamer 188

Die Komponente (b) ist im erfindungsgemäss verwendeten NanoFood in einer Konzentration von ca. 1 bis ca. 50 Gew.-%, bezogen auf das Gesamtgewicht der Komponenten (a), (b) und (c) vorhanden.

Das NanoFood, enthaltend
(a) ein membranbildendes Molekül,
(b) als Coemulgator ein Alkali-, Ammonium- oder Aminiumsalz, ein Salz der Gallensäure, ein Partialfettsäureester des Sorbitans, ein Zuckerester, ein Fettsäurepartialglycerid, ein Polyglycerinester von Fettsäuren, ein Propylenglycolester von Fettsäuren, ein Milchsäureester, ein Protein, ein Emulgator vom Polyoxyethylen Typ sowie Mischungen dieser Coemulgatoren und
(c) einen lipophilen Bestandteil,
stellt einen weiteren Erfindungsgegenstand dar.

Die Komponente (c) ist vorzugsweise ein natürliches, ein synthetisches oder ein partialsynthetisches Di- oder Triglycerid, ein Fett, ein wasserunlöslicher, ein schwer wasserlöslicher, ein öliger oder ein in Oel lösliche Nährstoff beziehungsweise Nutraceutical oder Mischungen dieser Stoffe.

Für die erfindungsgemässe Anwendung geeignete Nährstoffe sind Fette, Eiweisse, Kohlenhydrate, Salze, Vitamine und Spurenstoffe.

Für die erfindungsgemässe Anwendung geeignete Nutraceuticals sind beispielsweise Vitamine, Antioxidantien, Phytochemicals und Zoochemicals.

Beispiele von Vitaminen und Antioxidantien sind Vitamin E und Derivate, Tocotrienole, Vitmamin A und Derivate, Vitamin C, Beta-Carotin, Carotinoide, Flavonoide, Lycopene, Luteine und Liponsäuren.

Beispiele von Phytochemicals, d.h. natürliche Komponenten von Pflanzen, sind Oele und ätherische Oele wie z. B. Weizenkeimöl, Maiskeimöl, Distelöl, Leinöl, Hagebuttenkernöl, Hanföl, Haselnussöl, Heilbuttleberöl, Johannisbeersamenöl, Kümmelöl, Kürbiskernöl, Olivenöl, Erdnussöl, Rapsöl, Sojaöl, Sesamöl, Aprikosen- und Pfirsichkernöl, Baumwollsaatöl, Kirschkernöl, Pistazienkernöl, Nachtkerzenöl, Borretschöl, Reiskeimöl, Senfsaatöl, Sonnenblumenöl, Traubenkernöl, Pfefferminzöl, Melissenöl, Citronellöl, Lemongrasöl, Zitronenöl, Salbeiöl, ölige Thymianextrakte, Rosmarinöl, Orangenblütenöl, Pinen freies Wacholderöl, Kamillenöl und -extrakte, Schafgarbenblütenöl, Zimtöl, Korianderöl, Anisöl, und Fenchelöl.

Scharfstoffe, Bitterstoffe, Gewürze, Gerbstoffe und Saponine sind weitere Beispiele von Phytochemicals.

Beispiele von Zoochemicals, d.h. natürliche Bestandteile tierischen Gewebes sind Lebertran, Fischöl, Lachsöl, Lachsöl Ethylesterkonzentrat und Fischöl Ethylesterkonzentrat.

Weitere Beispiele von Nutraceuticals sind ungesättigte Omega-9-Fettsäuren wie z.B. Oelsäure, Omega-6-Fettsäuren wie z.B. Linolsäure, gamma-Linolensäure und Arachidonsäure und Omega-3-Fettsäuren wie z.B. alpha Linolensäure, Eicosapentaensäure und Docosahexaensäure.

Ebenfalls Beispiele von Nutraceuticals sind Aminosäuren, Carnitin, Creatin, Dithiolethione (Oltipraz), Tamoxifen, Reaveratrol und Taurin.

Die Komponente (c) ist im erfindungegemäss eingesetzten NanoFood in einer bevorzugten Konzentration von 0,1 bis 70 Gew.-%, bezogen auf das Gesamtgewicht der Komponenten (a), (b) und (c) vorhanden.

Gegebenenfalls enthält das erfindungsgemäss eingesetzte NanoFood als fakultative Komponente (d) einen Lösungsvermittler, wie z.B. Ethanol.

Eine NanoFood-Zusammensetzung mit den Komponenten (a), (b) und (c) zeichnet sich bei Dispersion im wässrigen Medium durch günstige Phaseneigenschaften aus. So ist bei vorhandener Opaleszenz und Transparenz im Gegenlicht nur an einer äusserst geringen milchigen Trübung zu erkennen, dass die Dispersion noch physikalische Unterschiede gegenüber dem Idealzustand einer echten molekularen Lösung aufweist. Elektronenmikroskopische Abbildungen zeigen, dass eine Population von mehr als 98 % in einer Gaussschen Verteilung als Suspension von Partikeln (Nanopartikel) mit einer Teilchengrösse kleiner als ca. 60 nm (Nanodispersion), typischerweise kleiner als ca. 30 nm, vorliegt. Diese Unterschiede gegenüber einer echten Lösung sind aber aufgrund der besonders guten Homogenitätseigenschaften der Dispersion tolerierbar, die beispielsweise an einer überraschend hohen Lagerstabilität, z. B. keine Entmischung nach mehrmonatiger Lagerung bei Temperaturen bis Raumtemperatur (durch Extrapolation zu erwartende Stabilität länger als zwei Jahre), nachweisbar sind.

Zur Herstellung der NanoFood werden die beiden Komponenten (b) und (c), gegebenenfalls unter Erwärmen zu einer homogenen flüssigen Phase gemischt. In dieser Phase wird die Komponente (a), gegebenenfalls unter Zuhilfenahme des Lösungsvermittlers Ethanol, gelöst. Daraus resultiert eine homogene Lösung. Diese wird der gegebenenfalls wasserlösliche Nährstoffe oder Nutraceuticals enthaltenden wässrigen Phase unter Rühren zugegeben. Dabei bilden sich die extrem homogenen NanoFood von einer mittleren Grösse < 60 nm, typischerweise < 30 nm. Laser-Lichtstreumessungen und elektronenmikroskopische Untersuchungen (Cryo-TEM) bestätigen die sehr kleine Grösse und hervorragende Homogenität dieser Nanopartikel.

Alternativ können NanoFood auch hergestellt werden, in dem Komponente (a) und/oder (b) in der wässrigen Phase vorgelegt werden, in die die Komponente (c) unter Rühren eingebracht wird.

Die NanoFood-Zusammensetzung wird erfindungsgemäss für Nahrungs-Endprodukte verwendet.

Vorzugsweise sind die Nahrungs-Endprodukte flüssig, halbfest oder fest.

Flüssige Nahrungs-Endprodukte sind z.B stille und kohlensäurehaltige Mineralwasser, aromatisierte Wasser, Frucht-, Obst-, Gemüsesäfte und Sirupe, Milch, Milch-Imitationsprodukte und Sossen.

Beispiele halbfester Nahrungs-Endprodukte sind Yoghurt, Quark, Margarine, hochfetthaltigen Emulsionen, Aufstriche und Speiseeis.

Feste Nahrungs-Endprodukte sind z. B. Instantprodukte, Schokolade, Brot und Kleingebäck, Fleisch und Wurstwaren, Teigwaren und Kartoffelprodukte.

Diese Nahrungs-Endprodukte, enthaltend NanoFood, bilden einen weiteren Erfindungsgegenstand.

Die Nahrungs-Endprodukte enthalten dabei in ihrer wässrigen Phase das NanoFood in einer Konzentration von 0.01 bis 60, vorzugsweise von 0.05 bis 30 Gew.%.

Zur Herstellung von flüssigen (Beispiele 10-15) und halbfesten (Beispiele 16-19) Nahrungs-Endprodukten werden die NanoFood in den wässrigen Anteil des Endproduktes eingearbeitet.

Feste Nahrungs-Endprodukte wie z.B. Instantprodukte werden durch Besprühen oder Tränken mit NanoFood gecoatet oder beladen.

Die Nahrungs-Endprodukte werden vorzugsweise zur Ernährung, zur diätetischen Ernährung, zur Stimulierung und Verbesserung von Körperfunktionen, zur Vorbeugung, zur unterstützenden Therapie oder zur Therapie von Krankheiten verwendet.

In den folgenden Beispielen beziehen sich die Prozentsätze auf das Gewicht. Die Mengen beziehen sich bei den eingesetzten Verbindungen, wenn nicht anders angegeben, auf die Reinsubstanz.

### NanoFood Herstellungsbeispiele:

| Beispiel 1: | |
|---|---|
| Soja Lecithin | 1.7 % |
| Polysorbat 80 | 3.4 % |
| Miglyol 812 | 3.4 % |
| Ethanol | 1.4 % |
| Wasser | ad 100.0 % |

Polysorbat und Miglyol werden gemischt. Zu der Mischung wird das Lacithin in Ethanol gelöst hinzugegeben. Daraus resultiert eine homogene Lösung, die unter Rühren dem Wasser zugesetzt wird.

| Beispiel 2: | |
|---|---|
| Soja Lecithin | 1.7 % |
| Vitamin E Polyethylene Glycol Succinate (Vitamin E TPGS, Eastman) | 3.4 % |
| Miglyol 812 | 3.4 % |
| Ethanol | 1.4 % |
| Wasser | ad 100.0 % |

| Beispiel 3: | |
|---|---|
| Soja Lecithin | 1.7 % |
| Sucrosemonolaurat | 3.4 % |
| Miglyol 812 | 3.4 % |
| Ethanol | 1.4 % |
| Wasser | ad 100.0 % |

| Beispiel 4: | |
|---|---|
| Vitamin E Acetat | 5.0 % |
| Soja Lecithin | 1.1 % |
| Polysorbat 80 | 3.8 % |
| Miglyol 812 | 1.8 % |
| Ethanol | 0.9 % |
| Wasser | ad 100.0 % |

| Beispiel 5: | |
|---|---|
| Vitamin A Palmitat | 1.8 % |
| Vitamin E Acetat | 0.1 % |
| Soja Lecithin | 1.7 % |
| Polysorbat 80 | 3.1 % |
| Miglyol 812 | 1.8 % |
| Ethanol | 1.4 % |
| Wasser | ad 100.0 % |

| Beispiel 6: | |
|---|---|
| Beta-Carotin | 0.05 % |
| Soja Lecithin | 1.70 % |
| Polysorbat 80 | 3.40 % |
| Miglyol 812 | 3.40 % |
| Ethanol | 1.40 % |
| Wasser | ad 100.0 % |

| Beispiel 7: | |
|---|---|
| Vitamin E Acetat | 0.1000 % |
| Vitamin A Palmitat | 0.0100 % |
| Vitamin C | 0.6000 % |
| Vitamin B1 | 0.0140 % |
| Vitamin B2 | 0.0160 % |
| Vitamin B6 | 0.0220 % |
| Vitamin B12 | 0.0003 % |
| Nicotinamid | 0.1800 % |
| Folsäure | 0.0040 % |
| Pantothensäure | 0.1000 % |
| Soja Lecithin | 1.7000 % |
| Polysorbat 80 | 3.1000 % |
| Miglyol 812 | 3.1000 % |
| Ethanol | 1.4000 % |
| Wasser | ad 100.0000 % |

| Beispiel 8: | |
|---|---|
| Nachtkerzenöl | 3.6 % |
| Soja Lecithin | 1.7 % |
| Polysorbat 80 | 3.4 % |
| Ethanol | 1.4 % |
| Wasser | ad 100.00 % |

| Beispiel 9: | |
|---|---|
| Fischöl | 5.0 % |
| Vitamin E Acetat | 0.2 % |
| Soja Lecithin | 2.0 % |
| Polysorbat 80 | 1.6 % |
| Wasser | ad 100.0 % |

### Beispiele für Nahrungs-Endprodukte mit NanoFood

Zur Herstellung untenstehender flüssiger oder halbfester Nahrungs-Endprodukte werden die NanoFoods in den wässrigen Anteil des Endproduktes eingearbeitet.

| Beispiel 10: Vitamin E Drink | |
|---|---|
| Zitronensäure | 0.4 % |
| Glucose | 7.5 % |
| Aroma | 0.1 % |
| NanoFood gemäss Beispiel 4 | ad 100.0 % |

| Beispiel 11: Multivitamin Drink | |
|---|---|
| Zitronensäure | 0.4 % |
| Glucose | 7.5 % |
| Aroma | 0.1 % |
| NanoFood gemäss Beispiel 7 | 10.0 % |
| Wasser | ad 100.0 % |

| Beispiel 12: Omega-3-Fettsäuren Drink | |
|---|---|
| Zitronensäure | 0.4 % |
| Glucose | 7.5 % |
| Aroma | 0.1 % |
| NanoFood gemäss Beispiel 9 | 10.0 % |
| Wasser | ad 100.0 % |

| Beispiel 13: Milch angereichert mit Vitamin E Acetat | |
|---|---|
| NanoFood gemäss Beispiel 4 | 10.0 % |
| Milch | ad 100.0 % |

| Beispiel 14: Multivitamin Mineralwasser | |
|---|---|
| NanoFood gemäss Beispiel 7 | 10.0 % |
| Mineralwasser | ad 100.0 % |

| Beispiel 15: Orangensaft angereichert mit Omega-3-Fettsäuren | |
|---|---|
| NanoFood gemäss Beispiel 9 | 10.0 % |
| Orangensaft | ad 100.0 % |

| Beispiel 16: Multivitamin Yoghurt | |
|---|---|
| NanoFood gemäss Beispiel 7 | 10.0 % |
| Yoghurt | ad 100.0 % |

| Beispiel 17: Yoghurt angereichert mit Omega-3-Fettsäuren | |
|---|---|
| NanoFood gemässe Beispiel 9 | 10.0 % |
| Yoghurt | ad 100.0 % |

| Beispiel 18: Yoghurtdrink angereichert mit gamma Linolsäure | |
|---|---|
| NanoFood gemäss Beispiel 8 | 10.0 % |
| Yoghurtdrink | ad 100.0 % |

| Beispiel 19: Multivitamin Yoghurtdrink | |
|---|---|
| NanoFood gemässe Beispiel 7 | 10.0 % |
| Yoghurtdrink | ad 100.0 % |

## Patentansprüche

1. Verwendung eines NanoFood, enthaltend als präparative Zusammensetzung
(a) ein membranbildendes Molekül,
(b) einen Coemulgator und
(c) einen lipophilen Bestandteil
in Nahrungs-Endprodukten.

2. Verwendung nach Anspruch 1, dadurch gekennzeichnet, dass das NanoFood enthält:
(a) als membranbildende Moleküle Substanzen, die geeignet sind, Zweischichtensysteme auszubilden,
(b) als Coemulgatoren Substanzen, die bevorzugt O/W Strukturen ausbilden und
(c) als lipophile Komponente ein lipophiler Nährstoff beziehungsweise lipophilies Nutraceutical.

3. Verwendung nach Anspruch 1 oder 2, dadurch gekennzeichnet, dass das NanoFood als Komponente
(a) ein natürliches Phospholipid, ein hydriertes oder teilhydriertes Phospholipid, ein Lysophospholipid oder Mischungen aus diesen enthält.

4. Verwendung nach einem der Ansprüche 1 bis 3, dadurch gekennzeichnet, dass die Komponente (a) im erfindungsgemäss verwendeten NanoFood in einer Konzentration von 0,1 bis 30 Gew.-%, bezogen auf das Gesamtgewicht der Komponenten (a), (b) und (c) vorhanden ist.

5. Verwendung nach einem der Ansprüche 1 bis 4, dadurch gekennzeichnet, dass das NanoFood als Komponente
(b) Alkalimetall-, Ammonium- oder Aminsalze von Fettsäuren, Alkylsulfate, Salze der Gallensäure, Partialfettsäureester des Sorbitans, Zuckerester, Fettsäurepartial-glyceride, Polyglycerinester von Fettsäuren, Propylenglycolester von Fettsäuren, Milchsäureester von Fettsäuren oder Proteine enthält.

6. Verwendung nach Anspruch 5, dadurch gekennzeichnet, dass das NanoFood als Komponente
(b) ein Zuckerester enthält.

7. Verwendung nach einem der Ansprüche 1 bis 4, dadurch gekennzeichnet, dass das NanoFood als Komponente
(b) Emulgatoren vom Polyoxyethlene Typ enthält.

8. Verwendung nach Anspruch 7, dadurch gekennzeichnet, dass das NanoFood als Komponente
(b) Polyethoxylierte Sorbitanfettsäureester, Polyethoxylierte Vitamin E Derivate, Polyethoxylierte Fettsäurepartialglyceride, Polyethoxilierte Kohlenhydrate oder Blockpolymerisate von Ethylenoxid und Propylenoxid enthalten.

9. Verwendung nach einem der Ansprüche 1 bis 8, dadurch gekennzeichnet, dass die Komponente (b) im erfindungsgemäss verwendeten NanoFood in einer Konzentration von 1 bis 50 Gew.-%, bezogen auf das Gesamtgewicht der Komponenten (a), (b) und (c) vorhanden ist.

10. Verwendung nach einem der Ansprüche 1 bis 9, dadurch gekennzeichnet, dass die Komponente
(c) ein natürliches, ein synthetisches oder ein partialsynthetisches Di- oder Triglycerid, ein Fett, ein wasserunlöslicher, ein schwer wasserlöslicher, ein öliger oder ein in Oel löslicher Nährstoff beziehungsweise Nutraceutical oder Mischungen dieser Stoffe ist.

11. Verwendung nach Anspruch 10, dadurch gekennzeichnet, dass das NanoFood als Komponente
(c) ein fettlösliches Vitamin, ein Carotinoid, ein Flavonoid, ein pflanzliches oder tierisches Oel, ein ätherische Oel, eine Omega-9-Fettsäure, eine Omega-6-Fettsäure oder eine Omega-3-Fettsäure enthält.

12. Verwendung nach einem der Ansprüche 1 bis 11, dadurch gekennzeichnet, dass die Komponente (c) im erfindungsgemäss eingesetzten NanoFood in einer Konzentration von 0,1 bis 70 Gew.-%, bezogen auf das Gesamtgewicht der Komponenten (a), (b) und (c), vorhanden ist.

13. Verwendung nach einem der Ansprüche 1 bis 11, dadurch gekennzeichnet, dass das Nahrungs-Endprodukt flüssig, halbfest oder fest ist.

14. Flüssige Nahrungs-Endprodukt in Form eines Mineralwassers, eines Frucht-, Obst- oder Gemüsesaftes, eines Sirups, einer Milch, eines Milch-Imitationsproduktes, enthaltend ein NanoFood wie in Anspruch 1 definiert.

15. Halbfeste Nahrungs-Endprodukt in Form eines Yoghurts, eines Quarks, einer Margarine, einer hochfetthaltigen Emulsion, eines Aufstriches, eines Speiseeises, eines Mineralwassers, enthaltend ein NanoFood wie in Anspruch 1 definiert.

16. Feste Nahrungs-Endprodukt in Form eines Instantpulvers, enthaltend ein NanoFood wie in Anspruch 1 definiert.

17. Nahrungs-Endprodukte nach einem der Ansprüche 14 bis 16, dadurch gekennzeichnet, dass das NanoFood in ihrer wässrigen Phase vorhanden ist.

18. Nahrungs-Endprodukte nach einem der Ansprüche 14 bis 17, dadurch gekennzeichnet, dass die wässrige Phase das NanoFood in einer Konzentration von 0,01 bis 60 Gew.-% enthält.

19. Nahrungs-Endprodukte nach Anspruch 18, dadurch gekennzeichnet, dass die wässrige Phase das NanoFood in einer Konzentration von 0,05 bis 20 Gew.-% enthält.

20. Nahrungs-Endprodukte nach Anspruch 14, dadurch gekennzeichnet, dass das NanoFood im Endprodukt per se vorhanden ist.

21. Verfahren zur Herstellung der flüssigen oder halbfesten Nahrungs-Endprodukte nach Anspruch 14 oder 15, dadurch gekennzeichnet, dass die NanoFood in den wässrigen Anteil der Endprodukte eingearbeitet werden.

22. Verfahren zur Herstellung der festen Nahrungs-Endprodukte nach Anspruch 16, dadurch gekennzeichnet, dass die Nahrungs-Endprodukte mit dem NanoFood überzogen oder beladen werden.

23. Verwendung der Nahrungs-Endprodukte nach einem der Ansprüche 13 bis 20 zur Ernährung, zur diätetischen Ernährung, zur Stimulierung und Verbesserung von Körperfunktionen, zur Vorbeugung, zur unterstützenden Therapie oder zur Therapie von Krankheiten.

24. Verwendung der Nahrungs-Endprodukte nach einem der Ansprüche 13 bis 20 als Getränk, als Nahrungsmittel, als Diätetikum, als Energy Drink, als Energy Food oder als Functional Food.

25. NanoFood, enthaltend
(a) ein membranbildendes Molekül,
(b) Alkalimetall-, Ammonium- oder Aminsalze von Fettsäuren, Alkylsulfate, Salze der Gallensäure, Partialfettsäureester des Sorbitans, Zuckerester, Fettsäurepartial-glyceride, Polyglycerinester von Fettsäuren, Propylenglycolester von Fettsäuren, Milchsäureester von Fettsäuren oder Proteine und
(c) einen lipophilen Bestandteil.

26. NanoFood, enthaltend
(a) ein membranbildendes Molekül,
(b) als Coemulgator einen Zuckerester und
(c) einen lipophilen Bestandteil.

27. NanoFood, enthaltend
(a) ein membranbildendes Molekül,
(b) als Coemulgator einen Emulgator vom Polyoxyethylen Typ und
(c) einen lipophilen Bestandteil.

28. NanoFood, enthaltend
(a) ein membranbildendes Molekül,
(b) als Coemulgator Polyethoxylierte Sorbitanfettsäureester, Polyethoxylierte Vitamin E Derivate, Polyethoxylierte Fettsäurepartialglyceride, Polyethoxilierte Kohlenhydrate, Blockpolymerisate von Ethylenoxid und Propylenoxid und
(c) einen lipophilen Bestandteil.

29. NanoFood nach einem der Ansprüche 25-28 dadurch gekennzeichnet das Komponente
(c) ein fettlösliches Vitamin, ein Carotinoid, ein Flavonoid, ein pflanzliches oder tierisches Oel, ein ätherische Oel, eine Omega-9-Fettsäure, eine Omega-6-Fettsäure oder eine Omega-3-Fettsäure enthält.
